# FASCICULE DE BREVET EUROPEEN

(11) **EP 4 704 935 B1**
(45) Date de publication et mention de la délivrance du brevet: **02.12.2026**
(21) Numéro de dépôt: 24729892.0
(22) Date de dépôt: 03.05.2024
(51) Int. Cl.: A61L 31/10, A61F 9/007, A61L 31/14

(54) **POLYMÈRES THERMOGÉLIFIANTS APTES À FORMER UN REVÊTEMENT GLISSANT POUR INJECTEUR OPHTALMIQUE**
THERMOGELIERENDE POLYMERE ZUR BILDUNG EINER GLEITBESCHICHTUNG FÜR EINEN OPHTHALMISCHEN INJEKTOR
THERMOGELLING POLYMERS ABLE TO FORM A SLIPPERY COATING FOR AN OPHTHALMIC INJECTOR

(30) Priorité: 05.05.2023 FR 2304533
(43) Date de publication de la demande: 11.03.2026
(73) Titulaire: Polymerexpert, 33600 Pessac (FR)
(72) Inventeur: DOLATKHANI, Marc, 33610 CESTAS (FR); PAGNOUX, Anne, 33114 LE BARP (FR)
(74) Mandataire: Santarelli
(86) Numéro de dépôt international: PCT/FR2024/050584
(87) Numéro de publication internationale: WO 2024/231629

(56) Documents cités:
- WO-A1-2022/112730
- FR-A1- 2 986 532

## Description

### Domaine technique

L'invention concerne l'élaboration d'un revêtement glissant destiné à revêtir au moins une partie d'un dispositif d'injection d'implant intraoculaire, en particulier l'embout et/ou la cartouche, permettant une injection par micro-incision. Ce revêtement a pour principale propriété de permettre à l'implant plié de glisser le long de l'embout et/ou la cartouche, tout en s'assurant qu'il ne se détache pas de son support et soit entrainé dans l'œil.

### Etat de la technique

Le remplacement du cristallin de l'œil affecté par la cataracte est assuré par des implants intraoculaires. La chirurgie utilisant la phacoémulsification, permet la destruction du cristallin naturel et son élimination par une petite incision. Il a été développé des implants en matériaux souples et pliables pouvant être insérés à l'aide de dispositifs d'injection à travers la micro-incision réalisée pour la phacoémulsification.

Le système d'injection est composé d'un corps tubulaire dans lequel coulisse le piston d'injection surmonté d'un embout de forme conique dont le diamètre diminue au fur et à mesure que l'on se rapproche de l'extrémité d'injection (l'embout).

Le chirurgien appuie sur le piston dont l'extrémité pousse l'implant ; ce dernier est contraint de plus en plus dans l'embout de l'injecteur et finit par ressortir de l'injecteur totalement plié. Il est ainsi possible d'injecter un implant de plus de 6mm de diamètre par une incision de taille inférieure à 3 mm. De très grandes contraintes sont alors exercées sur l'implant lors du transfert. Pour limiter la force d'injection et permettre à l'implant de sortir de l'embout sans dommages, il est nécessaire d'optimiser la géométrie de l'embout et la nature du support d'une part, et, d'utiliser d'autre part un « lubrifiant ».

Dans la suite de la description, on utilisera indifféremment les termes « dispositif d'injection d'implant intraoculaire », « système d'injection » ou « injecteur ophtalmique ».

Les demandes de brevet EP1173115, EP2344073 ou bien WO/2007 054644 et WO/2007 021412, par exemple, décrivent des systèmes d'injection avec des conceptions géométriques différentes. De nombreux injecteurs commercialement disponibles relatent les différentes formes et/ou inventions mécaniques décrites dans les brevets. On peut citer par exemple les injecteurs monoblocs (embouts et corps d'injection liés) de type Skyjet^{®} commercialisés par Carl Zeiss Meditec ou bien les injecteurs Accujet^{®}, Navijet^{®} ou Viscojet^{®} de Medicel AG où l'embout est amovible et est constitué d'une chambre d'injection dans laquelle on place l'implant qui est immédiatement plié et passe par une canule pour être injecté, ou bien les injecteurs de type Monarch^{®} des laboratoires Alcon où l'embout est amovible et l'implant est glissé dans l'embout sans pliage préalable.

Les caractéristiques mécaniques du matériau constitutif de l'embout ont également une influence sur l'injectabilité. En effet, le matériau thermoplastique utilisé pour la fabrication de l'embout doit permettre une certaine déformabilité pour accompagner les contraintes infligées à l'implant tout en présentant une bonne rigidité. De plus, les matériaux thermoplastiques utilisés doivent pouvoir être injectés avec des cadences élevées. Ces matériaux sont choisis, de préférence, dans la famille des polypropylènes, des polyamides, des polyuréthanes ou des polyesters et plus particulièrement dans la famille des polypropylènes et des polyamides.

Le choix du matériau de l'embout et/ou de la cartouche ainsi que l'optimisation de sa géométrie ne suffisent cependant pas pour injecter des implants via de micro-incisions de façon satisfaisante. Il est impératif d'utiliser un lubrifiant permettant à l'implant de glisser dans l'embout et/ou la cartouche. Alternativement, l'embout et la cartouche peuvent être solidaires et former un seul élément. Cette alternative est couverte par l'expression « l'embout et/ou la cartouche » ou « embout/cartouche ». Plusieurs approches différentes permettant le glissement sont décrites dans la littérature.

La première concerne l'utilisation d'un agent de migration (en anglais « blooming agent ») intégré par compoundage dans le matériau thermoplastique. Il s'agit d'une molécule organique tensioactive de faible masse molaire de type monostéarate de glycérol (GMS) qui est compoundé avec le polypropylène ou bien le polyamide. Il est réparti uniformément dans le support thermoplastique juste après injection et finit par migrer à la surface du support après plusieurs jours, voire plusieurs semaines. Ce phénomène de migration est lié à la petite taille de la molécule tensio-active qui est mobile comparée aux chaînes macromoléculaires. A titre d'exemple, le brevet US6733507 décrit des cartouches en polypropylène contenant un agent lubrifiant qui migre à la surface par phénomène de migration (« blooming »).

Cette approche présente deux inconvénients majeurs.

Le premier est la présence de traces blanches sur les implants injectés. Elles sont dues à l'agent de migration qui n'est pas lié à la surface de l'embout et qui est entraîné lors de l'injection. En effet, les agents de migration utilisés ne sont pas hydrosolubles et leur élimination n'est possible qu'après de nombreux rinçages une fois l'implant injecté.

Le second inconvénient est lié au phénomène de migration de l'agent de migration à la surface du support. Cette migration peut durer plusieurs jours, voire plusieurs semaines, en fonction des conditions de mise en œuvre (injection de la pièce), de la température de stockage, de post-traitement, des conditions de stérilisation, etc... avant qu'une quantité suffisante de lubrifiant se trouve à la surface de la cartouche d'injection. La qualité de la lubrification va donc dépendre du temps d'attente entre la fabrication de l'injecteur et l'utilisation de celui-ci par le praticien. Si cette période est trop courte, la lubrification n'est pas assurée de manière satisfaisante et si elle est trop longue, l'implant est couvert de taches blanches (présence de l'agent de migration) lors de l'injection. Pour pallier cet inconvénient, la demande WO2005/018505 propose de traiter thermiquement les pièces afin d'accélérer le phénomène de migration et d'obtenir un état quasi-stable (quantité de lubrifiant suffisante à la surface du support) permettant des injections reproductibles. Une autre voie décrite par le brevet US7348038 suggère un traitement plasma pour assurer une liaison physique de l'agent de migration à la cartouche.

La non-reproductibilité de la lubrification et la présence de taches blanchâtres sur les implants lors de l'injection représentent les principaux défauts de l'utilisation des agents de migration.

Pour éviter ces inconvénients, la demande WO2013/083935 décrit une composition polymère métastable comprenant un mélange d'au moins un polymère constitutif et au moins un copolymère de fonction partiellement miscibles ou compatibles, dans laquelle
- ledit polymère constitutif est un polymère thermoplastique présentant une température de transition vitreuse (Tg) ou une température de fusion (Tf) supérieure ou égale à 80°C, et est présent dans le mélange en proportion massique comprise entre 85% et 99,5%, et
- ledit copolymère de fonction a une température de transition vitreuse (Tg) ou une température de fusion (Tf) supérieure ou égale à 40°C, possède au moins 60% massique d'unités monomères à caractère hydrophile, et des séquences ou blocs hydrophobes partiellement miscibles ou compatibles avec le polymère constitutif. Cette composition permet la fabrication d'un dispositif biomédical présentant une surface glissante sans nécessiter le revêtement d'un matériau. Le principe repose sur la migration du copolymère de fonction au sein du polymère constitutif, qui nécessite l'utilisation d'un copolymère de fonction de masse molaire faible, capable de migrer à la surface du polymère constitutif.

Dans la demande FR2986532, il est mentionné que le but recherché diffère totalement de l'approche « revêtement » (p. 16, l.8-15):
« La présente invention *diffère totalement de l'approche* « revêtement » (« coating »). Cette dernière, qui consiste à déposer un film polymère à la surface du dispositif ou de certains de ses éléments, nécessite de réaliser plusieurs opérations dont *l'activation de la surface, la dépose du polymère en solution et l'évaporation du solvant. L'approche proposée dans la présente invention permet d'éviter toutes ces* étapes dans le processus de fabrication et conduit à une simplification du procédé à *la seule étape de mise en œuvre par injection.* »

Ainsi, dans la demande FR2986532, la migration du polymère métastable est quasi immédiate après l'opération de mise en œuvre de l'embout ce qui assure une reproductibilité des propriétés glissantes. De plus, les interactions fortes entre le polymère métastable et le polymère thermoplastique constitutif de l'embout permettent une très bonne accroche et empêchent le polymère métastable d'être entrainé dans l'œil au moment de l'injection de l'implant.

Une autre approche permettant le glissement des implants dans les embouts/cartouches des injecteurs est la mise en place d'un revêtement hydrophile à l'intérieur de l'embout. Le principe de lubrification consiste à gonfler le revêtement hydrophile par l'addition d'un produit visqueux (solution d'hyaluronate ou bien d'hydroxypropylméthyl cellulose) et à glisser ainsi sur un film d'eau formé à l'interface.

Les demandes de brevet ou brevets JP5690838, JP3254752, US5716364, EP1949871, WO96/22062, WO2007/030009, WO2010/118080, US7687097, US7348038, WO2010/059655, par exemple, décrivent la possibilité de réaliser un revêtement permettant de diminuer ou éliminer la friction entre l'implant et la cartouche.

Le revêtement hydrophile est lié soit par des liaisons chimiques covalentes soit par des liaisons physiques à la surface de l'embout et/ou cartouche.

En ce qui concerne les revêtements liés par des interactions physiques au support, une activation de la surface par un plasma ou corona ou bien d'autres méthodes photochimiques, est systématiquement préconisée comme présenté, par exemple, dans les demandes de brevet ou brevets JP5690838, JP3254752, EP1949871,

US 5716364, WO2010/059655, WO2010/118080 ou bien US7348038. Une fois la surface activée, le polymère en solution est déposé et le solvant est ensuite évaporé. Les polymères hydrophiles sont sélectionnés parmi les polyacide acrylique, polyacide méthacrylique, polyvinylacétate, polyacrylamide, polyvinylpyrolidone et leur copolymère, et parfois un mélange de plusieurs de ces polymères.

Cependant, il arrive que les polymères constitutifs du revêtement se détachent du support ou se solubilisent par le produit visqueux et soient entraînés par l'implant dans l'œil. Si le praticien ne rince pas de manière satisfaisante l'œil après la mise en place de l'implant, les résidus du revêtement peuvent à terme causer de l'inflammation.

Il est par ailleurs décrit des revêtements liés de manière covalente à la cartouche et/ou à l'embout, notamment dans les demandes de brevets ou brevets US7687097 et WO96/22062. L'approche préconisée consiste à immerger la cartouche prétraitée ou non par plasma dans un précurseur présentant des fonctions réactives, par exemple des groupements acrylates, puis ensuite par voie radicalaire (thermique ou irradiation UV) amorcer la polymérisation du précurseur dont certaines chaînes viennent réagir avec les radicaux formés au niveau de la surface de la cartouche. D'autres demandes de brevets ou brevets tels que US6238799, US6866936 ou bien WO96/23602 décrivent le greffage covalent de polymères hydrophiles, voire d'hydrogels, sur des supports de dispositifs médicaux. Le principe est basé sur l'élaboration de réseaux interpénétrés constitués d'un polyuréthane associé à un autre polymère hydrophile.

Cependant, le greffage covalent de revêtement à la surface de l'embout et/ou cartouche de l'injecteur ophtalmique est une opération longue et coûteuse qui limite l'intérêt de l'approche.

Il est donc recherché d'élaborer un revêtement glissant qui ne nécessite pas un greffage chimique à la surface de l'embout et/ou de la cartouche de l'injecteur et qui ne se détache pas lors de l'injection de l'implant.

La demande WO2022/112730 décrit l'utilisation d'un polymère apte à former un revêtement glissant pour injecteur ophtalmique . Les fortes masses molaires associées à une balance hydrophile-hydrophobe ajustée du polymère constitutif du revêtement confèrent à celui-ci de bonnes propriétés glissantes sans se détacher et sans être entrainé dans l'œil lors de l'injection de l'implant.

La demande EP1521795 décrit des polymères thermosensibles capables de former des gels thermoréversibles dans l'eau. Ces polymères sont formés de chaînes linéaires de copolymère tribloc polyoxyéthylène-polyoxypropylène-polyoxyéthylène avec des groupes organiques liés à une ou aux deux extrémités via des liaisons carbamate et urée. La demande EP1521795 a également pour objet leur utilisation dans des compositions thérapeutiques ou non thérapeutiques, en particulier cosmétiques. Leur utilisation en tant que revêtement (sous forme de film) d'un dispositif médical n'est cependant pas décrite dans cette demande.

### Exposé de l'invention

L'invention concerne l'utilisation de polymères thermosensibles aptes à former des gels thermoréversibles pour former un revêtement glissant sur au moins une partie d'un dispositif d'injection d'implant intraoculaire.

En particulier, l'invention concerne l'utilisation de polymères formés de chaînes linéaires de copolymère tribloc polyoxyéthylène-polyoxypropylène-polyoxyéthylène avec des groupes organiques liés à une ou aux deux extrémités via des liaisons carbamate et/ou urée, tels que décrits dans la demande EP1521795 en tant que revêtement de la surface interne d'une ou plusieurs parties d'un dispositif d'injection d'implant intraoculaire, permettant de lui conférer un caractère glissant, ainsi que le procédé de revêtement de ladite surface, permettant la bonne adhésion du polymère. L'invention a également pour objet une partie d'un dispositif d'injection d'implant intraoculaire, dont la surface interne est revêtue d'un polymère tel que défini ci-dessus, ainsi qu'un dispositif d'injection d'implant intraoculaire comprenant au moins une partie dont la surface interne est revêtue par ledit polymère.

### Brève description des figures

La Figure 1 représente la caractérisation rhéologique en écoulement (10 s⁻¹) entre 10 °C et 50°C du polymère de l'exemple 1 en solution dans l'eau à une concentration de 7,5%.
La Figure 2 représente la caractérisation rhéologique en écoulement (10 s⁻¹) entre 10 °C et 50°C du polymère de l'exemple 2 en solution dans l'eau à une concentration de 7,5%.

### Description détaillée

La présente invention a pour objectif de proposer un revêtement glissant pour dispositifs d'injection d'implant intraoculaire, ledit revêtement étant élaboré à partir de polymères thermogélifiants à LCST (en anglais « Lower Crititcal Solubilty Temperature »).

Il s'agit de polymères thermosensibles hydrosolubles apte à former des gels physiques thermoréversibles à haut indice de viscosification, qui comprennent à la fois des enchaînements comprenant au moins une chaîne linéaire de type polyoxyalkylène triblocs thermosensible constituée de blocs de polyoxyde d'éthylène (POE) et de blocs de polyoxyde de propylène (POP), ladite chaîne étant de la forme POE-POP-POE et étant allongée à au moins une de ses extrémités par un groupement organique via une liaison carbamate et/ou une liaison urée.

L'invention a donc pour objet l'utilisation des polymères thermosensibles hydrosolubles décrits ci-dessus pour former un revêtement glissant sur au moins une partie d'un dispositif d'injection d'implant oculaire, en particulier sur la surface interne de ladite partie.

On entend par « gel physique thermoréversible », un réseau polymère dont les liaisons inter chaînes sont assurées par des liaisons non-covalentes, en l'occurrence dans ce cas, par des liaisons Hydrogène.

On entend par « polymère à haut indice de viscosification », un polymère, qui en solution (en l'occurrence dans ce cas, en solution dans l'eau) permet à la solution d'atteindre des viscosités élevées à savoir plus de 10 Pa.s sous 10 s⁻¹ de cisaillement pour une concentration en polymère de 7,5%.

Ledit polymère présente en solution dans l'eau à une concentration inférieure à 10%, une faible viscosité à température ambiante et est apte à former des gels physiques à haut indice de viscosification à une température supérieure à 25°C (thermogélification).

Ledit polymère est formé de parties hydrophobes thermosensibles et de parties hydrophiles. La formation du gel s'explique par l'auto-association de portions thermosensibles en micro-domaines hydrophobes, l'ensemble du polymère étant maintenu en solution par les segments hydrophiles.

Lesdits polymères sont suffisamment hydrophiles pour être gonflés par les produits visqueux utilisés lors de la chirurgie de l'œil (gel d'hyaluronate de sodium, hydroxy propyl méthyl cellulose) tout en étant suffisamment hydrophobes pour éviter toute solubilisation à la température corporelle. En effet, la thermogélification à partir de 25°C crée des interactions physiques fortes entre les chaines polymères conduisant à la formation d'un gel physique non soluble dans l'eau. Cette réticulation physique évite toute dispersion involontaire dudit revêtement dans l'œil du patient, évitant ainsi des problèmes post-opératoires.

Ainsi, selon l'un de ses aspects, l'invention concerne l'élaboration d'un revêtement pour injecteur ophtalmique à partir d'un polymère dont la balance hydrophile-hydrophobe et la capacité de thermogélifier à partir de 25°C, conduisant à un gel physique, sont parfaitement adaptées pour former un film à la surface d'un embout d'injecteur ophtalmique.

Ce film présente également la spécificité d'être glissant grâce à l'hydrophilie du polymère, d'une part, et d'être parfaitement résistant à l'eau grâce à son caractère thermogélifiant (formation d'un réseau physique non soluble dans l'eau), d'autre part. Cette résistance à l'eau évite tout entrainement du polymère dans l'œil et assure une bonne adhérence à l'injecteur ophtalmique qui est au contact de l'œil et dont la température au moment de l'injection dans l'œil est supérieure à 25°C. Contrairement à la demande WO2022/112730, dans laquelle le polymère doit avoir une masse molaire suffisamment élevée (masse molaire moyenne en nombre entre 20 000 g/mol et 1 000 000 g/mol et de préférence entre 50 000 g/mol et 500 000 g/mol), pour éviter sa solubilisation dans l'eau lors de son utilisation, le polymère décrit plus haut peut présenter des masses molaires bien plus faibles à condition qu'il soit thermogélifiant. Cette thermogélification doit être basée sur la LCST du polymère pour permettre la formation d'un réseau physique. La formation de ce gel physique évite la solubilisation du revêtement en milieu aqueux lors de l'injection de l'implant. Le caractère « glissant » du revêtement selon l'invention peut être mesuré, par exemple, en utilisant un injecteur ophtalmique monobloc entièrement fabriqué par injection/moulage à partir de ladite composition ou bien un injecteur constitué de deux pièces dans lequel seuls la cartouche de chargement et l'embout sont fabriqués à partir de ladite composition. On mesure la force de poussée sur le piston de l'injecteur nécessaire pour éjecter l'implant chargé dans la cartouche de l'injecteur, à travers un embout dont le diamètre de sortie est inférieur à 3 mm, par exemple 2 mm. Cette mesure peut être effectuée par compression au moyen d'un dynamomètre de type Instron 3367 équipé d'un capteur de force de sensibilité 0,5 kN à une vitesse de 4 mm/s et à température ambiante. On considère que la propriété de glissement est importante pour des valeurs de force comprises entre 5 et 10 N. Elle est modérée pour des valeurs de force supérieures à 10 N et inférieures à 15 N, et faible pour des valeurs de force supérieures à 15 N.

Ainsi, on considère que le dépôt d'un revêtement selon l'invention confère à un injecteur ophtalmique un caractère glissant lorsqu'un praticien injecte sans dommage un implant d'une dioptrie inférieure ou égale à 30D dans l'œil d'un patient, en appliquant une force inférieure ou égale à 20N.

L'invention concerne également le procédé de mise en œuvre du revêtement. Le procédé selon l'invention permet la bonne adhésion dudit polymère sur la surface de l'embout et/ou de la cartouche de l'injecteur pour éviter que ledit revêtement ne soit décroché et emporté par l'implant, lors de son injection, dans l'œil du patient.

Selon l'invention, ledit revêtement est déposé à la surface de l'embout et/ou de la cartouche de l'injecteur. Son accrochage est essentiellement assuré par les blocs hydrophobes (POP) dudit polymère qui présentent une très bonne compatibilité chimique avec la matière constitutive de l'embout et/ou de la cartouche de l'injecteur. Les blocs hydrophiles (POE) dudit polymère, qui ont peu ou pas d'interactions avec la surface de l'embout et/ou de la cartouche de l'injecteur, permettent d'obtenir les propriétés glissantes souhaitées.

L'un des objectifs de cette invention est d'élaborer un polymère présentant des blocs hydrophobes présentant des interactions suffisamment fortes avec la surface de l'embout et/ou de la cartouche de l'injecteur (sans qu'il y ait interpénétration entre les chaînes polymères) et des blocs hydrophiles permettant d'obtenir des propriétés glissantes suffisantes pour l'injection d'implants intraoculaires.

Avantageusement, le revêtement glissant selon l'invention présente les propriétés suivantes :
- une balance hydrophile - hydrophobe ajustée,
- un caractère thermogélifiant à partir de 25°C conduisant à son insolubilité dans l'eau,
- des propriétés filmogènes,
- une bonne affinité avec le support, par exemple en polypropylène ou bien en polyamide.

Selon un aspect de l'invention, le polymère constitutif du revêtement selon l'invention peut être choisi parmi la famille des polyuréthanes thermogélifiants, notamment ceux décrits dans la demande EP1521795. L'ajustement de la balance hydrophile - hydrophobe de ce type de polymère peut être obtenu par le choix de la nature du polymère triblocs POE-POP-POE et des di-isocyanates.

L'invention concerne donc, selon l'un de ses aspects, l'utilisation d'un polymère tel que défini plus haut consistant en une pluralité de chaînes linéaires de type polyoxyalkylène tribloc POE-POP-POE reliées entre elles par des liaisons carbamate et/ou urée issues de la réaction des extrémités hydroxyle du polyoxyalkylène triblocs POE-POP-POE avec un di-isocyanate.

Les chaines dudit polymère peuvent être terminées par ajout d'éthanol ou allongées par l'addition d'un monométhyléther de polyéthylène glycol.

Le polymère selon l'invention comprend, de préférence, une partie hydrophile majoritaire, de préférence au moins 70% massique d'unités monomères hydrophiles de type POE.

Les polyuréthanes possèdent également de bonnes propriétés filmogènes ; ils sont, d'ailleurs, utilisés comme vernis pour de nombreuses applications. Enfin, leur caractère polaire et leur capacité à créer des liaisons hydrogène leur permettent de présenter une grande affinité avec de nombreux supports.

L'invention concerne donc, selon l'un de ses aspects, l'utilisation d'un polymère consistant en une pluralité de chaînes linéaires de type polyoxyalkylène triblocs POE-POP-POE, de formule (I) : 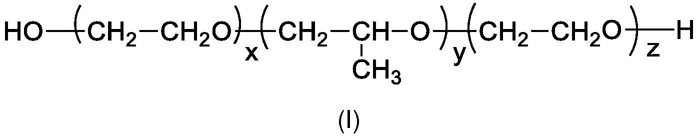
dans laquelle, 10< x <150, 10< y <150, 10< z <150,
lesdites chaînes étant reliées entre elles par des liaisons carbamate et/ou urée issues de la réaction des extrémités hydroxyle du polyoxyalkylène triblocs POE-POP-POE avec un di-isocyanate,
pour former un revêtement glissant sur au moins une partie d'un dispositif d'injection d'implant oculaire, en particulier sur la surface interne de ladite partie. Préférentiellement, x est égal z. Préférentiellement 10< x=z< 150 et 15<y<60.

Les di-isocyanates susceptibles d'être utilisés pour la synthèse desdits polymères peuvent notamment être choisis parmi les composés suivants :

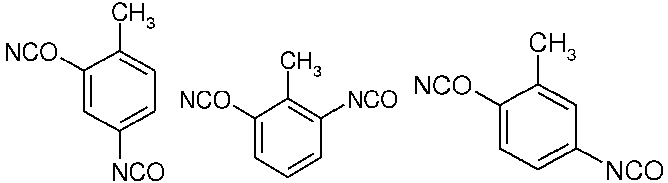

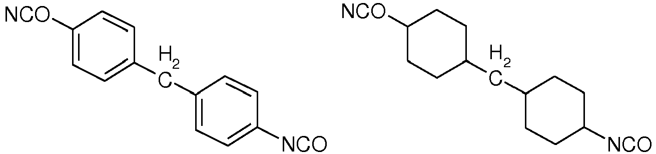

Un di-isocyanate préféré est, par exemple, le diisocyanate-4,4'de dicyclohexylméthane.

Les polymères thermogélifiants aptes à former un revêtement glissant pour injecteur ophtalmique peuvent être préparés comme décrit dans la demande EP1521795. Brièvement, le procédé comprend la réaction d'au moins un polymère linéaire de type polyoxyalkylène triblocs thermosensible de formule (I) présentant au moins une fonction hydroxy terminale avec au moins une molécule organique porteuse d'au moins une fonction isocyanate de façon à les relier ensemble par des liaisons carbamate ou urée.

De préférence, ledit procédé d'obtention comprend la réaction du polyoxyalkylène triblocs POE-POP-POE avec un di-isocyanate, de telle sorte que le polymère présente au moins 70% massique de POE.

Les chaînes de polyuréthane du polymère de formule ainsi obtenu peuvent être soit terminées par ajout d'éthanol, soit allongées par l'addition d'un monométhyléther de polyéthylène glycol comprenant entre 2 et 70 unités oxyde d'éthylène, de manière à former les polymères de formule (II) ci-dessous : 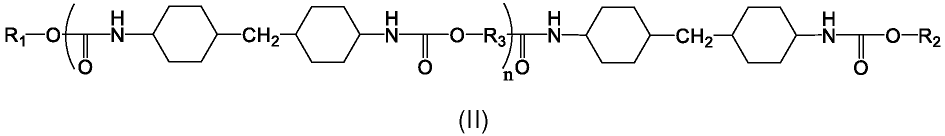 dans laquelle :
- R₁ et R₂, identiques ou différents, représentent

   -(CH₂-CH₂-O)ₚ-CH₃ ou -CH₂-CH₃ :
- n est un nombre entier de 2 à 50 et p est un nombre entier de 2 à 70 ;
- R₃ représente la formule (III) 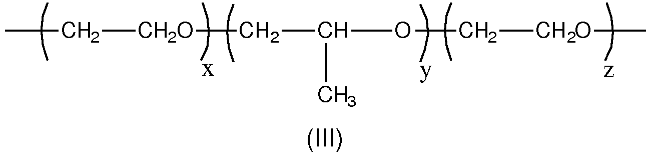
dans laquelle x, y et z sont tels que définis ci-dessus pour la formule (I).

L'utilisation des polymères de formule (II) pour former un revêtement glissant sur au moins une partie d'un dispositif d'injection d'implant intraoculaire est un objet de l'invention.

Des composés utilisables aux fins de l'invention sont également, par exemple, ceux des exemples 1 et 2 de la demande EP1521795.

Le polymère thermogélifiant apte à former un revêtement glissant selon l'invention présente en solution dans l'eau à une concentration inférieure à 10% une faible viscosité à température ambiante et est apte à former des gels physiques à haut indice de viscosification à une température supérieure à 25°C. En particulier, ladite faible viscosité peut être inférieure à 1000 mPa.s, notamment de 0,1 à 500 mPa.s, par exemple de 0,1 à 100 mPa.s.

Un aspect avantageux du polymère constitutif du revêtement selon l'invention est sa capacité à former un film. En effet, le caractère glissant du revêtement est également lié à la formation d'un film lisse et homogène. Il a été également trouvé que les interactions inter et intra-chaînes créées lors de l'élévation de la température (phénomène de thermogélification) contribuent très fortement à la formation d'un film très cohésif. Cette réticulation physique du polymère rend le film insoluble dans l'eau. Ainsi, ce dernier n'est pas entraîné dans l'œil lors de l'injection de l'implant. L'invention concerne également un procédé de revêtement de la surface interne d'une ou plusieurs parties d'un dispositif d'injection d'implant intraoculaire , comprenant les étapes suivantes :
- soumettre la surface interne de la ou des partie(s) du dispositif d'injection d'implants intraoculaires à revêtir, en particulier l'embout et/ou la cartouche, à un traitement fortement oxydant, et
- déposer une solution de polymère tel que défini plus haut.

De préférence, ledit polymère consiste en une pluralité de chaînes linéaires de type polyoxyalkylène tribloc POE-POP-POE reliées entre elles par des liaisons carbamate et/ou urée issues de la réaction des extrémités hydroxyle du polyoxyalkylène triblocs POE-POP-POE avec un di-isocyanate, et dans lequel ladite chaine POE-POP-POE répond à la formule (I) 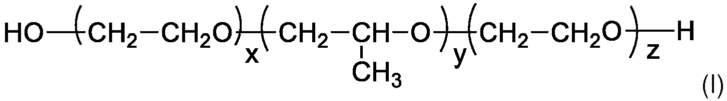 dans laquelle, 10< x <150, 10< y <150, 10< z <150.

Ainsi, un aspect ultérieur de l'invention concerne, en particulier, un procédé de dépôt du film polymère sur la matière constitutive de l'embout et/ou cartouche de l'injecteur ophtalmique, qui permet avantageusement d'éviter le décrochage du revêtement et son entrainement dans l'œil du patient.

De préférence, pour assurer une bonne adhésion du revêtement sur l'embout et/ou la cartouche de l'injecteur ophtalmique, on peut, par exemple, procéder à un traitement spécifique de ce dernier. Dans un premier temps, on élimine toute trace de dépôts, de type graisse ou particules, à la surface de l'embout et /ou de la cartouche par lavage, par exemple par un lavage à l'alcool suivi d'un rinçage à l'eau pure. La surface du matériau peut ensuite être soumise à un traitement permettant une forte oxydation, afin d'augmenter la tension de surface du support. Ainsi, l'embout et/ou la cartouche peuvent être traité(s) au corona, au plasma à l'argon, l'oxygène ou à l'air. La présence de fonctions créées grâce à l'oxydation à la surface de l'embout et/ou la cartouche permet la création aisée de liaisons de type hydrogène avec le revêtement. En effet, la présence systématique de liaisons uréthane du revêtement assure une très bonne adhésion sur le support traité par le corona, ou le plasma à l'argon, l'oxygène ou à l'air, notamment grâce aux liaisons hydrogène.

Le revêtement mettant en œuvre le polymère thermogélifiant apte à former un revêtement glissant selon l'invention décrit plus haut se fait de préférence par voie solvant. Ledit polymère est solubilisé à une concentration allant de 0,5% à 3% en masse dans un solvant du polymère, et de préférence dans un mélange constitué d'eau et d'éthanol. L'embout et/ou cartouche est(sont) rempli(s) par cette solution. Après quelques secondes, par exemple environ 3 secondes, l'excédent est évacué ; le polymère déposé à la surface de l'embout et/ou cartouche est alors séché. Le film ainsi déposé est lisse et homogène.

Les essais d'injection réalisés avec ces embouts/cartouches revêtus selon les différents aspects de l'invention ont permis une injection facile des implants, y compris par de petits diamètres (<3mm) sans détachement ou solubilisation du revêtement. Aucune trace du polymère constitutif du revêtement n'a pu être trouvé par analyse HPLC, aussi bien sur la surface des implants que dans le milieu dans lequel les implants ont été injectés.

Les aspects généraux et particuliers de l'invention décrits plus haut s'appliquent indifféremment à l'utilisation des polymères thermogélifiants décrits plus haut pour former un revêtement glissant sur au moins une partie d'un dispositif d'injection d'implant intraoculaire qu'au procédé de revêtement de la surface interne d'au moins une partie d'un dispositif d'injection d'implant intraoculaire les mettant en œuvre.

### Exemples

### Exemple 1 : Préparation d'un polyuréthane thermogélifiant

Le polyuréthane thermogélifiant est préparé dans un réacteur équipé de :
- un système d'agitation efficace (de type ancre et bi-étagé)
- une alimentation par pompe péristaltique pour l'introduction des isocyanates
- une arrivée d'azote
- une sortie sur un bulleur
d'un orifice libre pour l'introduction de l'Adekanol^{®} F108 et du PEG600.

Les réactifs utilisés sont présentés dans le tableau 1.

**[Tableau 1]**

| | Rapport Molaire | Masse molaire (g/mol) | Indice OH (mg KOH/g) | Masse (g) | Aspect |
|---|---|---|---|---|---|
| Adekanol^{®} F108 | 1 | - | I_{OH}=6 à 8* | 100_{±}1% | Pastille |
| Diisocyanate-4,4'de dicyclohexylméthane | 2,06 | 262 | - | 100x(I_{OH}/207,5) ±1% | liquide |
| monométhyl ether de polyéthylène glycol (PEG 600) | 1,73 | 600 | - | 100x(I_{OH}/107,9) ±1% | liquide à 25 °C |
| Carboxylate de bismuth | 175 ppm/F108 | | - | 0,0175±0,0005 | liquide |
| Ethanol 99% absolu | 0,1mL/100 g F108 | | - | 0,1 mL | liquide |

| | | | | | |
|---|---|---|---|---|---|
| * valeur à relever sur le CA de l'ADEKANOL^{®} F108 fourni par la société ADEKA | | | | | |

La synthèse comprend 3 phases :

### A) Séchage du poloxamer et du PEG600

Les opérations suivantes sont effectuées le jour de la synthèse.

### 1) Introduire l'Adekanol^{®} F108 dans le réacteur principal.

Porter à 120°C tout en maintenant le réacteur sous vide dynamique à au moins 50 mbars et sous agitation de façon continue. Lorsque la matière est totalement fondue, maintenir le chauffage et le vide pendant encore 2 à 4 h (absence de bulles dans le milieu). Maintenir le vide entre 20h et 24h au maximum. Une grande partie de l'eau contenue dans l'Adekanol^{®} F108 doit ainsi être retirée.

2) Placer le PEG600 dans un autre réacteur permettant le chauffage et l'application du vide à au moins 40mbars. Faire fondre sous vide à 80 °C et sous agitation continue le PEG600 et maintenir le système ainsi 2h à partir de la fusion complète du PEG600.

### B) Chargement des réactifs dans le réacteur principal

3) Ajouter le catalyseur (carboxylate de bismuth) sous flux d'azote sur l'Adekanol^{®} F108 préalablement séché. Mélanger fortement.

4) 15 min après l'ajout du catalyseur, ajouter le diisocyanate-4,4'de dicyclohexylméthane en 15 min.

5) Effectuer un prélèvement 1h après le début de l'ajout des isocyanates, si la conversion des fonctions isocyanate est supérieure ou égale à 58%, introduire le PEG600.

Lorsque la conversion est atteinte, ajouter en une seule fois le PEG600 sec et liquide (80°C), le tout sous flux d'azote (réacteur de synthèse et réacteur de PEG600).

6) Maintenir sous agitation pendant 2h.

7) Lorsqu'au moins 99% des isocyanates sont consommés, ajouter l'éthanol au cœur du milieu réactionnel. Agiter pendant 30 min avant de récupérer le polymère.

8) Vérifier l'absence des isocyanates, et procéder à 4 cycles de vide/azote.

### C) Récupération du polymère

Le polymère est coulé à chaud. Le rendement de cette synthèse est supérieur ou égal à 97%.

Après refroidissement, il est broyé sous forme de poudre. Cette poudre peut ensuite être solubilisé dans de l'acétone et reprécipitée dans un hydrocarbure comme le pentane ou l'heptane.

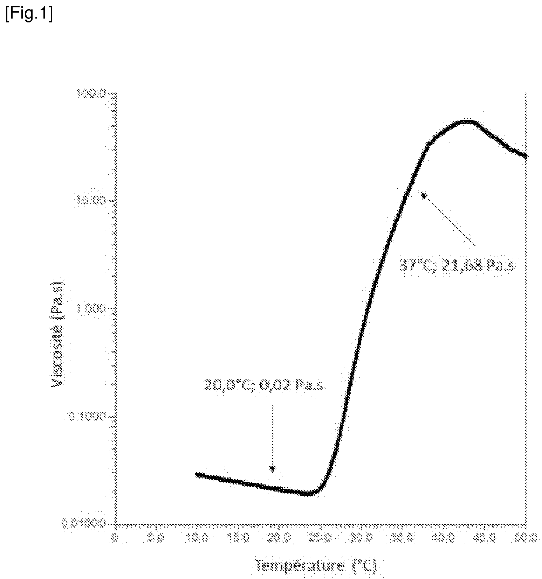

La Figure 1 représente la caractérisation rhéologique en écoulement (10s⁻¹) en fonction de la température, entre 10 °C et 50 °C, du polymère de l'exemple 1 en solution dans l'eau à une concentration de 7,5% et confirme bien son caractère thermogélifiant.

### Exemple 2 : Polyuréthane thermogélifiant

Le polyuréthane thermogélifiant est préparé dans un réacteur équipé de :
- un système d'agitation efficace (de type ancre et bi-étagé)
- une alimentation par pompe péristaltique pour l'introduction des isocyanates
- une arrivée d'azote
- une sortie sur un bulleur
- un orifice libre pour l'introduction du Pluronic^{®} F127 et du PEG600 .

Les réactifs utilisés sont présentés dans le tableau 2.

**[Tableau 2]**

| | Rapport Molaire | Masse molaire (g/mol) | Masse (g) | Nombre de moles x 10⁻³ | Aspect |
|---|---|---|---|---|---|
| Pluronic^{®} F127 | 1 | 13 000 | 100±1% | 7,692 | poudre hygroscopique |
| Diisocyanate-4,4'de dicyclohexylméthane | 2,06 | 262 | 4,152±1% | 15,846 | liquide |
| monométhyl ether du polyéthylène glycol (PEG 600) | 1,84 | 600 | 8,506±1% | 13,976 | Liquide à 25 °C |
| Carboxylate de bismuth | | | 0,047±1% | | liquide |
| Ethanol 99% absolu | | | 0,1 mL | | liquide |

La synthèse comprend 3 phases :

### A) Séchage du poloxamer et du PEG600

Les opérations suivantes sont effectuées le jour de la synthèse.

### 1) Introduire le Pluronic^{®} F127 dans le réacteur principal.

Porter à 120°C tout en maintenant le réacteur sous vide dynamique à au moins 50 mbars et sous agitation de façon continue. Lorsque la matière est totalement fondue, maintenir le chauffage et le vide pendant encore 2 à 4 heures (absence de bulles dans le milieu). Maintenir le vide entre 20h et 24h au maximum. Une grande partie de l'eau contenue dans le Pluronic^{®}F127 ainsi être retirée.

2) Placer le PEG600 dans un autre réacteur permettant le chauffage et l'application du vide à au moins 40mbars. Faire fondre sous vide à 80 °C et sous agitation continue le PEG600 et maintenir le système ainsi 2 heures à partir de la fusion complète du PEG600.

### B) Chargement des réactifs dans le réacteur principal

3) Ajouter le catalyseur (carboxylate de bismuth) sous flux d'azote sur le F127 préalablement séché. Mélanger fortement.

4) 15 minutes après l'ajout du catalyseur, ajouter le diisocyanate-4,4' de dicyclohexylméthane en 15 min.

5) Effectuer un prélèvement 1h après le début de l'ajout des isocyanates, si la conversion des fonctions isocyanate est supérieure ou égale à 58%, introduire le PEG600.

Lorsque la conversion est atteinte, ajouter en une seule fois le PEG600 sec et liquide (80°C), le tout sous flux d'azote (réacteur de synthèse et réacteur de PEG600).

6) Maintenir sous agitation pendant 2h.

7) Lorsqu'au moins 99% des isocyanates sont consommés, ajouter l'éthanol au cœur du milieu réactionnel. Agiter pendant 30 min avant de récupérer le polymère.

8) Vérifier l'absence des isocyanates, et procéder à 4 cycles de vide/azote.

### C) Récupération du polymère

Le polymère est coulé à chaud. Le rendement de cette synthèse est supérieur ou égal à 97%.

Après refroidissement, il est broyé sous forme de poudre. Cette poudre peut ensuite être solubilisé dans de l'acétone et reprécipitée dans un hydrocarbure comme le pentane ou l'heptane.

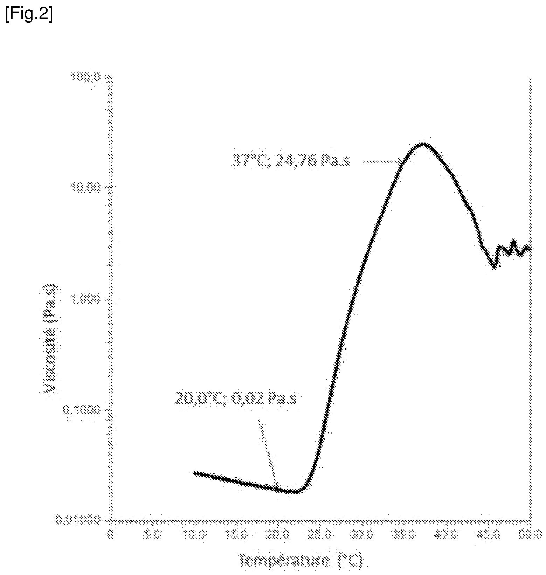

La Figure 2 représente la caractérisation rhéologique en écoulement (10s⁻¹) en fonction de la température, entre 10 °C et 50 °C, du polymère de l'exemple 2 en solution dans l'eau à une concentration de 7,5% et confirme bien son caractère thermogélifiant.

### Exemple 3 : traitement des embouts et cartouches avant application du revêtement selon l'invention

Les embouts/cartouches sont lavés 2 fois à l'éthanol puis rincés 2 fois à l'eau pure. Ils sont ensuite séchés à l'air comprimé.

L'appareil utilisé pour le plasma est un PlasmaNet^{®} MWGO. Le gaz utilisé est l'air à une pression de 0,4 mbar. Le temps d'utilisation du gaz est de 20 s et le traitement plasma dure 195 s à une puissance de 3000 W.

La tension de surface des embouts/cartouches est déterminée grâce à des encres testeurs après le traitement plasma afin de s'assurer de son efficacité. En effet, en fin de cycle, des échantillons d'embouts/cartouches sont prélevés ; de l'encre ACOTEST^{®} 56 mN/m est déposée à la surface des supports. Le traitement plasma est validé si l'encre s'étale parfaitement.

### Exemple 4 : application du revêtement selon l'invention

Le polymère de l'exemple 1 est solubilisé à 0,625% dans l'eau pendant au moins 48 h à température ambiante. La solution est ensuite filtrée sur un filtre de 0,8 µm. La solution est prête pour application.

Les embouts/cartouches des injecteurs de type Accujet^{®} sont remplis avec la solution préparée ci-dessus. L'excédent est évacué sous vide après environ 3 s de contact. Le dépôt est ensuite séché. Le film formé est lisse et homogène.

### Exemple 5 : essais d'injection

50 implants hydrophiles et 50 implants hydrophobes de dioptrie 28 ont été injectés à l'aide d'un injecteur disposant d'un embout de 2 mm ou de 2,6 mm de diamètre (traité selon le protocole de l'exemple 4).

Les essais d'injection réalisés, au moyen d'un injecteur de type Accujet^{®}, avec les revêtements élaborés à partir de différents polymères sont rapportés dans le tableau 3 ci-dessous. Ces essais sont réalisés en plongeant l'embout à un bain d'eau pure à 32°C pour se rapprocher au mieux des conditions d'injection dans l'œil humain.

La mesure a été effectuée par compression au moyen d'un dynamomètre de type Instron 3367 équipé d'un capteur de force de sensibilité 0,5 kN à une vitesse de 4 mm/s et à température ambiante.

**[Tableau 3]**

| **Référence** | **Lentille** | **Diamètre d'embout** | **Nature du revêtement** | **Force IOL sortie (N)** |
|---|---|---|---|---|
| | **Nature / Dioptrie** | | | |
| DIM22104 A | Hydrophobe* / 28 | 2,6 mm | Exemple 1 | 15 |
| | | | | 15 |
| | | | Moyenne | 15 |
| | | | Ecart-type | 0 |
| DIM22104 B | Hydrophobe* / 28 | 2,6 mm | Exemple 2 | 14 |
| | | | | 13 |
| | | | | 14 |
| | | | Moyenne | 14 |
| | | | Ecart-type | 1 |
| DIM22103 A | Hydrophile / 28 | 2,0 mm | Exemple 1 | 11 |
| | | | | 11 |
| | | | Moyenne | 11 |
| | | | Ecart-type | 0 |
| DIM22103 B | Hydrophile / 28 | 2,0 mm | Exemple 2 | 10 |
| | | | | 11 |
| | | | | 10 |
| | | | Moyenne | 10 |
| | | | Ecart-type | 1 |

| | | | | |
|---|---|---|---|---|
| *les lentilles choisies sont des implants Innoloop^{®} de la société Innolens Les résultats montrent que le revêtement déposé confère à l'injecteur un caractère glissant permettant d'injecter un implant intraoculaire d'au moins 6 mm de diamètre par un embout de 2 mm de diamètre pour les implants hydrophiles et de 2,6 mm pour les implants hydrophobes. | | | | |

### Exemple 6 : tenue du revêtement glissant

50 implants hydrophiles de dioptrie 28 ont été injectés à l'aide d'un injecteur disposant d'un embout de 2 mm de diamètre (traité selon le protocole de l'exemple 4)dans les conditions décrites à l'exemple 5.

Le bain d'eau contenant les implants a été concentré au rotavapor puis lyophilisé. Le résiduel a été analysé par HPLC (eau/acétonitrile). Aucune trace du revêtement mettant en œuvre le polymère de l'exemple 1 n'a été trouvée dans le produit analysé.

Les résultats montrent que le revêtement selon l'invention adhère à la surface de l'injecteur et ne se détache pas lors de l'injection de l'implant.

## Revendications

1. Utilisation d'un polymère thermosensible hydrosoluble formant des gels physiques thermoréversibles à haut indice de viscosification, ledit polymère comprenant à la fois des enchaînements comprenant au moins une chaîne linéaire de type polyoxyalkylène triblocs thermosensible constituée de blocs de polyoxyde d'éthylène (POE) et de blocs de polyoxyde de propylène (POP), ladite chaîne étant de la forme POE-POP-POE et étant allongée à au moins une de ses extrémités par un groupement organique via une liaison carbamate et/ou une liaison urée, pour former un revêtement glissant sur au moins une partie d'un dispositif d'injection d'implant intraoculaire.

2. Utilisation selon la revendication 1 pour le revêtement de la surface interne d'au moins une partie d'un dispositif d'injection d'implant intraoculaire.

3. Utilisation selon l'une des revendications 1 ou 2, dans laquelle ladite au moins une partie est l'embout et/ou la cartouche d'un injecteur ophtalmique.

4. Utilisation selon l'une quelconque des revendications 1 à 3, dans laquelle ledit polymère consiste en une pluralité de chaînes linéaires de type polyoxyalkylène tribloc POE-POP-POE reliées entre elles par des liaisons carbamate et/ou urée issues de la réaction des extrémités hydroxyle du polyoxyalkylène triblocs POE-POP-POE avec un di-isocyanate.

5. Utilisation l'une quelconque des revendications 1 à 3, dans laquelle ledit polymère consiste en une pluralité de chaînes linéaires de type polyoxyalkylène tribloc POE-POP-POE reliées entre elles par des liaisons carbamate et/ou urée issues de la réaction des extrémités hydroxyle du polyoxyalkylène triblocs POE-POP-POE avec un di-isocyanate, lesdites chaines étant soit terminées par ajout d'éthanol, soit allongées par l'addition d'un monométhyléther de polyéthylène glycol.

6. Utilisation selon l'une quelconque des revendications 1 à 5, dans laquelle ladite chaine POE-POP-POE répond à la formule (I) 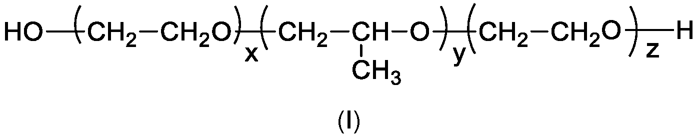 dans laquelle 10< x <150, 10< y <150 et 10< z <150.

7. Utilisation selon l'une quelconque des revendications 4 à 6, dans laquelle le di-isocyanate est choisi parmi :

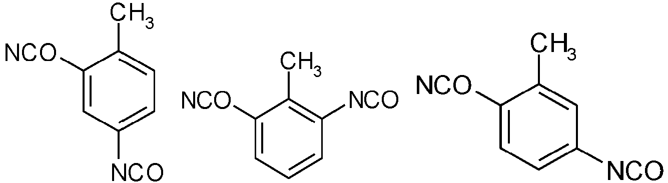

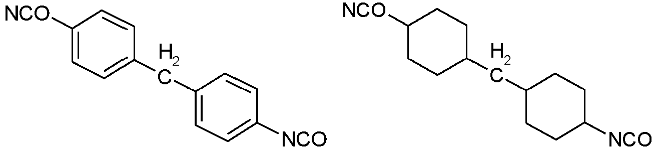

8. Utilisation selon l'une quelconque des revendications 5 à 7, dans laquelle ledit polymère répond à la formule (II) 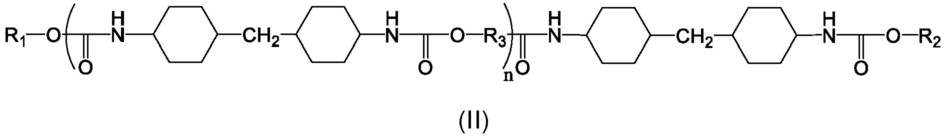 dans laquelle :
- R₁ et R₂, identiques ou différents, représentent
- (CH₂-CH₂-O)ₚ-CH₃ ou -CH₂-CH₃;
- n est un nombre entier de 2 à 50 et p est un nombre entier de 2 à 70 ;
- R₃ représente la formule (III) 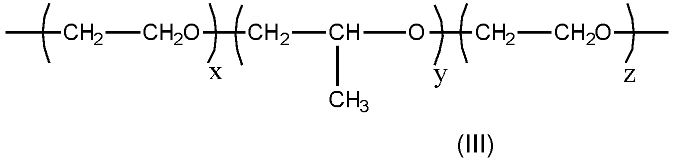 dans laquelle 10< x <150, 10< y <150 et 10< z <150.

9. Utilisation selon l'une des revendications 6 ou 8, dans laquelle, dans la formule (I) ou (III), x = z.

10. Utilisation selon l'une quelconque des revendication 6, 8 ou 9, dans laquelle, dans la formule (I) ou (III), 10< x <150, 15< y <60 et 10< z <150.

11. Utilisation selon l'une quelconque des revendications 1 à 10, dans laquelle ledit polymère présente en solution dans l'eau à une concentration inférieure à 10% une faible viscosité à température ambiante et est apte à former des gels physiques à haut indice de viscosification à une température supérieure à 25°C.

12. Procédé de revêtement de la surface interne d'une ou plusieurs parties d'un dispositif d'injection d'implant intraoculaire, comprenant les étapes suivantes :
- soumettre la surface interne de la ou des partie(s) du dispositif d'injection d'implants intraoculaires à revêtir, en particulier l'embout et/ou la cartouche, à un traitement fortement oxydant, et
- déposer une solution de polymère tel que défini dans l'une quelconque des revendications 1 à 11.

13. Procédé selon la revendication 12, dans lequel ledit polymère consiste en une pluralité de chaînes linéaires de type polyoxyalkylène tribloc POE-POP-POE reliées entre elles par des liaisons carbamate et/ou urée issues de la réaction des extrémités hydroxyle du polyoxyalkylène triblocs POE-POP-POE avec un di-isocyanate, et dans lequel ladite chaine POE-POP-POE répond à la formule (I) 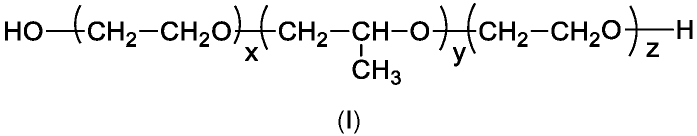 dans laquelle, 10< x <150, 10< y <150, 10< z <150.

14. Partie d'un dispositif d'injection d'implant intraoculaire, dont la surface interne est revêtue d'un polymère tel que défini dans l'une quelconque des revendications 1 à 11.

15. Dispositif d'injection d'implant intraoculaire comprenant au moins une partie dont la surface interne est revêtue d'un polymère tel que défini dans l'une quelconque des revendications 1 à 11.

## Patentansprüche

1. Verwendung eines wärmeempfindlichen, wasserlöslichen Polymers, das thermoreversible physikalische Gele mit hohem Viskositätsindex bildet, wobei das Polymer sowohl Verkettungen umfasst, die mindestens eine wärmeempfindliche lineare Triblock-Polyoxyalkylenkette umfassen, die aus Polyethylenoxidblöcken (POE) und Polypropylenoxidblöcken (POP) besteht, wobei die Kette in der Form POE-POP-POE vorliegt und an mindestens einem ihrer Enden durch eine organische Gruppe über eine Carbamatverbindung und/oder Harnstoffverbindung verlängert ist, um eine Gleitbeschichtung auf mindestens einem Teil einer Injektionsvorrichtung für ein intraokulares Implantat zu bilden.

2. Verwendung nach Anspruch 1 zur Beschichtung der inneren Oberfläche mindestens eines Teils einer Injektionsvorrichtung für intraokulare Implantate.

3. Verwendung nach einem der Ansprüche 1 oder 2, wobei der mindestens eine Teil die Spitze und/oder die Kartusche eines ophthalmischen Injektors ist.

4. Verwendung nach einem der Ansprüche 1 bis 3, wobei das Polymer aus einer Vielzahl von linearen POE-POP-POE-Triblock-Polyoxyalkylenketten besteht, die durch Carbamat- und/oder Harnstoffverbindungen miteinander verbunden sind, die aus der Reaktion der Hydroxylenden des POE-POP-POE-Triblock-Polyoxyalkylens mit einem Diisocyanat resultieren.

5. Verwendung nach einem der Ansprüche 1 bis 3, wobei das Polymer aus einer Vielzahl von linearen POE-POP-POE-Triblock-Polyoxyalkylenketten besteht, die durch Carbamat- und/oder Harnstoffverbindungen miteinander verbunden sind, die aus der Reaktion der Hydroxylenden des POE-POP-POE-Triblock-Polyoxyalkylens mit einem Diisocyanat resultieren, wobei die Ketten entweder durch Zugabe von Ethanol abgeschlossen oder durch Zugabe von Polyethylenglykol-Monomethylether verlängert werden.

6. Verwendung nach einem der Ansprüche 1 bis 5, wobei die POE-POP-POE-Kette der Formel (I) entspricht, 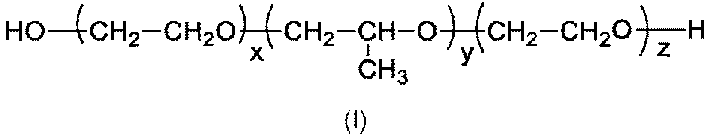 wobei 10< x <150, 10< y <150 und 10< z <150.

7. Verwendung nach einem der Ansprüche 4 bis 6, wobei das Diisocyanat ausgewählt ist aus:

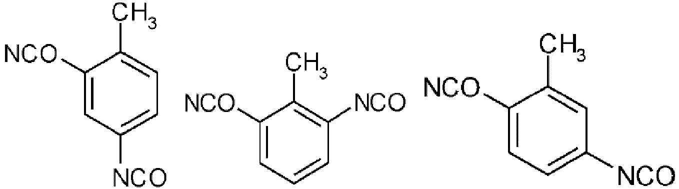

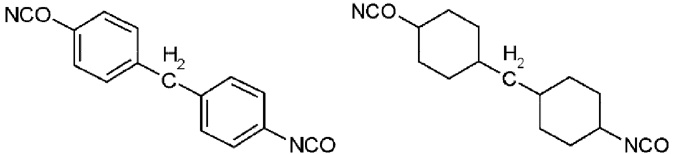

8. Verwendung nach einem der Ansprüche 5 bis 7, wobei das Polymer der Formel (II) entspricht, 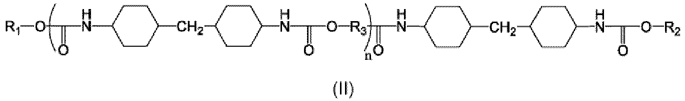 wobei:
- R₁ und R₂, gleich oder unterschiedlich,
-(CH₂-CH₂-O)ₚ-CH₃ oder -CH₂-CH₃ darstellen;
- n eine Ganzzahl von 2 bis 50 ist und p eine Ganzzahl von 2 bis 70 ist;
- R₃ die Formel (III) darstellt, 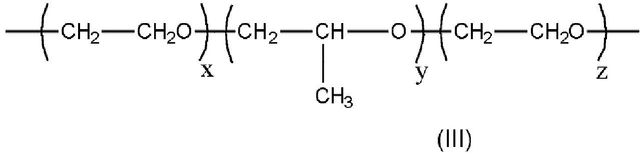 wobei 10< x <150, 10< y <150 und 10< z <150.

9. Verwendung nach einem der Ansprüche 6 oder 8, wobei in der Formel (I) oder (III), x = z.

10. Verwendung nach einem der Ansprüche 6, 8 oder 9, wobei in der Formel (I) oder (III), 10< x <150, 15< y <60 und 10< z <150.

11. Verwendung nach einem der Ansprüche 1 bis 10, wobei das Polymer in Wasserlösung in einer Konzentration von weniger als 10 % eine niedrige Viskosität bei Raumtemperatur aufweist und zur Bildung von physikalischen Gelen mit hohem Viskositätsindex bei einer Temperatur von mehr als 25 °C geeignet ist.

12. Verfahren zur Beschichtung der inneren Oberfläche eines oder mehrerer Teile einer Injektionsvorrichtung für intraokulare Implantate, umfassend die folgenden Schritte:
- Unterziehen der Innenfläche des/der zu beschichtenden Teil(e) der Injektionsvorrichtung für intraokulare Implantate, insbesondere der Spitze und/oder der Kartusche, einer stark oxidierenden Behandlung, und
- Einbringen einer Polymerlösung gemäß einem der Ansprüche 1 bis 11.

13. Verfahren nach Anspruch 12, wobei das Polymer aus einer Vielzahl von linearen POE-POP-POE-Triblock-Polyoxyalkylenketten besteht, die durch Carbamat- und/oder Harnstoffverbindungen miteinander verbunden sind, die aus der Reaktion der Hydroxylenden des POE-POP-POE-Triblock-Polyoxyalkylens mit einem Diisocyanat resultieren, und wobei die POE-POP-POE-Kette der Formel (I) entspricht, 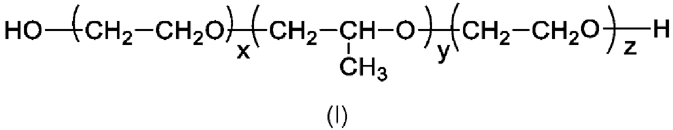 wobei, 10 < x <150, 10 < y < 150, 10 < z < 150.

14. Teil einer Injektionsvorrichtung für intraokulare Implantate, deren Innenfläche mit einem Polymer gemäß einem der Ansprüche 1 bis 11 beschichtet ist.

15. Injektionsvorrichtung für intraokulare Implantate, umfassend mindestens einen Teil, dessen Innenfläche mit einem Polymer gemäß einem der Ansprüche 1 bis 11 beschichtet ist.

## Claims

1. Use of a water-soluble heat-sensitive polymer forming thermoreversible physical gels with a high viscosity index, said polymer comprising both chain formations comprising at least one heat-sensitive polyoxyalkylene triblock type linear chain consisting of polyethylene oxide (PEO) blocks and polypropylene oxide (PPO) blocks, said chain having the form PEO-PPO-PEO and being extended at at least one of its ends by an organic group via a carbamate bond and/or a urea bond, to form a slippery coating on at least one part of an intraocular implant injection device.

2. Use according to claim 1 for coating the inner surface of at least one part of an intraocular implant injection device.

3. Use according to any one of claims 1 or 2, wherein said at least one part is the tip and/or the cartridge of an ophthalmic injector.

4. Use according to any one of claims 1 to 3, wherein said polymer consists in a plurality of PEO-PPO-PEO triblock polyoxyalkylene type linear chains connected to each other by carbamate and/or urea bonds obtained from reacting the hydroxyl ends of PEO-PPO-PEO triblock polyoxyalkylene with a diisocyanate.

5. Use of any one of claims 1 to 3, wherein said polymer consists in a plurality of PEO-PPO-PEO triblock polyoxyalkylene type linear chains connected to each other by carbamate and/or urea bonds obtained from reacting the hydroxyl ends of PEO-PPO-PEO triblock polyoxyalkylene with a diisocyanate, said chains being either terminated by adding ethanol or extended by adding a polyethylene glycol monomethyl ether.

6. Use according to any one of claims 1 to 5, wherein said PEO-PPO-PEO chain complies with formula (I) 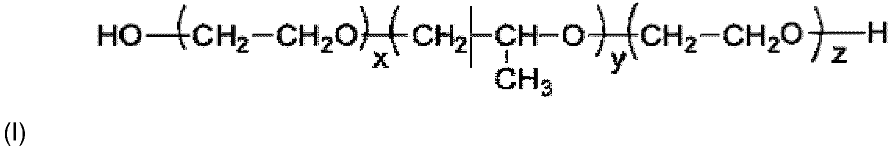 wherein 10< x <150, 10< y <150 and 10< z <150.

7. Use according to any one of claims 4 to 6, wherein the diisocyanate is chosen from:

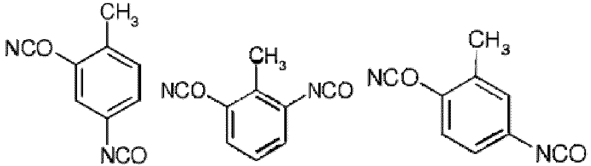

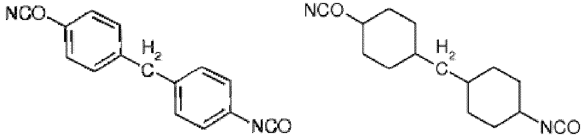

8. Use according to any one of claims 5 to 7, wherein said polymer complies with formula (II) 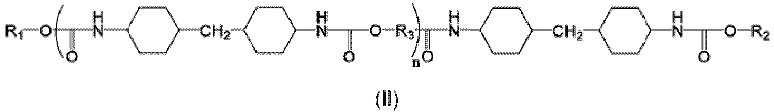 wherein:
- R₁ and R₂, identical or different, represent
-(CH₂-CH₂-O)ₚ-CH₃ or -CH₂-CH₃;
- n is an integer from 2 to 50 and p is an integer from 2 to 70
- R₃ represents formula (III) 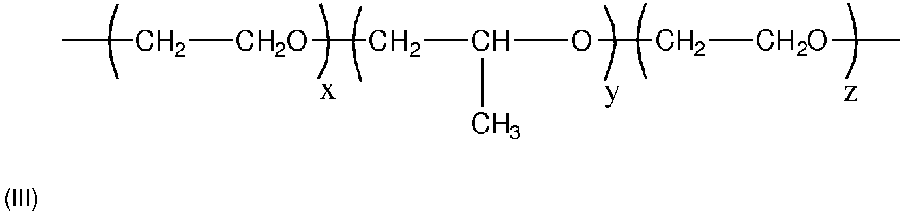 wherein 10< x <150, 10< y <150 and 10< z <150.

9. Use according to any one of claims 6 or 8, wherein, in formula (I) or (III), x = z.

10. Use according to any one of claims 6, 8 or 9, wherein, in formula (I) or (III), 10< x <150, 15< y <60 and 10< z <150.

11. Use according to any one of claims 1 to 10, wherein said polymer has a low viscosity at ambient temperature in solution in water at a concentration of less than 10% and is capable of forming physical gels with a high viscosity index at a temperature greater than 25°C.

12. Method for coating the inner surface of one or more parts of an intraocular implant injection device, comprising the following steps:
- subjecting the inner surface of the part(s) of the intraocular implant injection device to be coated, in particular the tip and/or the cartridge, to a strongly oxidizing treatment, and
- depositing a solution of polymer as defined in any one of claims 1 to 11.

13. Method according to claim 12, wherein said polymer consists in a plurality of PEO-PPO-PEO triblock polyoxyalkylene type linear chains connected to each other by carbamate and/or urea bonds obtained from reacting the hydroxyl ends of PEO-PPO-PEO triblock polyoxyalkylene with a diisocyanate, and wherein said PEO-PPO-PEO chain complies with formula (I) 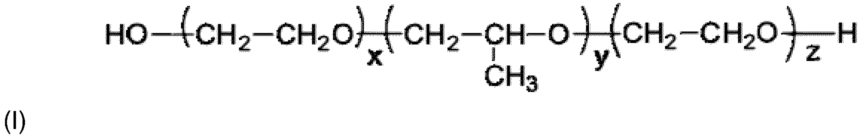 wherein, 10< x <150, 10< y <150, 10< z <150.

14. Part of an intraocular implant injection device, the inner surface of which is coated with a polymer as defined in any one of claims 1 to 11.

15. Intraocular implant injection device comprising at least one part, the inner surface of which is coated with a polymer as defined in any one of claims 1 to 11.
